(19) **Europäisches Patentamt**
European Patent Office
Office européen des brevets

(11) **EP 1 931 215 B3**

(12) **NEUE EUROPÄISCHE PATENTSCHRIFT**
Nach dem Beschränkungsverfahren (B3-1)

(45) Hinweis auf die Patenterteilung:
**04.11.2009 Patentblatt 2009/45**

(45) Veröffentlichungstag und Bekanntmachung des Hinweises auf die Entscheidung über den Antrag auf Beschränkung:
**B3-1 08.10.2014 Patentblatt 2014/41**

(21) Anmeldenummer: **06793374.7**

(22) Anmeldetag: **08.09.2006**

(51) Int Cl.:
*A23K 1/16* *(2006.01)*      *A23K 1/00* *(2006.01)*
*A23K 1/165* *(2006.01)*      *C12P 13/08* *(2006.01)*
*C07C 229/26* *(2006.01)*      *C12N 1/20* *(2006.01)*

(86) Internationale Anmeldenummer:
**PCT/EP2006/066192**

(87) Internationale Veröffentlichungsnummer:
**WO 2007/042363 (19.04.2007 Gazette 2007/16)**

(54) **L-LYSIN ENTHALTENDE FUTTERMITTELADDITIVE**

FEEDSTUFF ADDITIVE CONTAINING L-LYSINE

ADDITIFS D'ALIMENTS POUR ANIMAUX CONTENANT DE LA L-LYSINE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **08.10.2005 DE 102005048315**
**06.04.2006 DE 102006016158**

(43) Veröffentlichungstag der Anmeldung:
**18.06.2008 Patentblatt 2008/25**

(73) Patentinhaber: **Evonik Degussa GmbH**
**45128 Essen (DE)**

(72) Erfinder:
• **LOTTER, Hermann**
**63674 Altenstadt (DE)**
• **BECKER, Ulrich**
**63579 Freigericht-Horbach (DE)**
• **DÜBNER, Frank**
**61169 Friedberg (DE)**
• **KAEPPKE, Friederike**
**63571 Gelnhausen (DE)**
• **POHLISCH, Joachim**
**63571 Gelnhausen (DE)**
• **KELLE, Ralf**
**33330 Gütersloh (DE)**
• **SANDER, Cory M.**
**Blair, NE 68008 (US)**
• **FOSDICK, Lawrence Edward**
**Oskaloosa, Iowa 52577 (US)**

(56) Entgegenhaltungen:
**EP-A- 1 067 192      EP-A1- 1 062 877**
**DD-A1- 200 655      DE-B- 1 056 082**
**US-A- 5 876 780**

• **DATABASE WPI Week 199014 Derwent Publications Ltd., London, GB; AN 1990-105964 XP002405721 & SU 1 509 018 A (GORKI AGRIC INST) 23. September 1989 (1989-09-23)**
• **DATABASE WPI Week 198842 Derwent Publications Ltd., London, GB; AN 1988-298329 XP002405722 & SU 1 386 144 A (ODESS AGRIC INST) 7. April 1988 (1988-04-07)**

EP 1 931 215 B3

**Beschreibung**

Die Erfindung betrifft hellere und thermisch stabilere granulierte Tierfuttermitteladditive auf Fermentationsbrühebasis mit einem hohen Gehaft an L-Lysin und verlustärmere Verfahren zur Herstellung aus durch Fermentation erhaltenen Brühen.

Stand der Technik

Tierfuttermittel werden mit einzelnen Aminosäuren entsprechend dem Bedarf der Tiere supplementiert. Zur Supplementierung von Tierfuttermitteln, z. B. mit L-Lysin, wird bisher weit überwiegend das L-Lysin-monohydrochlorid mit einem L-Lysin-Gehalt von 78 % eingesetzt. Da das L-Lysin durch Fermentation hergestellt wird, muß es zur Herstellung des Monohydrochlorids zunächst einmal von allen übrigen Bestandteilen der rohen Fermentationsbrühe in aufwendigen Verfahrensschritten abgetrennt, dann in das Monohydrochlorid umgewandelt und letzteres zur Kristallisation gebracht werden. Dabei fallen eine große Anzahl von Nebenprodukten und die zur Aufarbeitung notwendigen Reagentien als Abfall an. Da eine hohe Reinheit des Tierfuttermittelsupplements nicht immer notwendig ist und zudem in den Nebenprodukten der Fermentation oft noch nutritiv wirksame Wertstoffe enthalten sind, hat es daher in der Vergangenheit nicht an Versuchen gefehlt, die aufwendige Herstellung von Futter-Aminosäuren, insbesondere von reinem L-Lysin-monohydrochlorid zu vermeiden und die rohe Fermentationsbrühe kostengünstiger in ein festes Tierfuttermittel zu überführen.

Als gravierender Nachteil hat sich die komplexe Zusammensetzung solcher Medien erwiesen, denn diese sind generell nur schlecht zu trocknen, dann hygroskopisch, praktisch nicht rieselfähig, verklumpungsgefährdet, und für die technisch anspruchsvolle Verarbeitung in Mischfutterwerken nicht geeignet. Dies trifft vor allem auf L-Lysin enthaltende Fermentationsprodukte zu. Die einfache Entwässerung der rohen Fermentationsbrühe durch Sprühtrocknung führte zu einem staubigen, stark hygroskopischen und nach kurzer Lagerzeit klumpigen Konzentrat, das in dieser Form nicht als Tierfuttermittel eingesetzt werden kann.

Die EP 0 533 039 betrifft Verfahren zur Herstellung eines Aminosäure-Tierfuttermittelsupplements auf Fermentationsbrühebasis, wobei das Supplement direkt durch Sprühtrocknung aus der Fermentationsbrühe gewonnen werden kann. Hierzu wird bei einer Variante ein Teil der Biomasse vor der Sprühtrocknung abgetrennt. Durch eine sehr saubere Führung der Fermentation, d. h. bei Erhalt einer an organischen Substanzen rückstandsarmen Fermentationsbrühe, kann die Brühesogarohne die Biomasse und ohne zusätzlichen Trägerhilfsstoff zu einem handhabbaren Granulat getrocknet werden.

Ca. 20 Gew.-% L-Lysin enthaltende feste Konzentrate sind aus der GB 1 439121 bekannt, in der auch L-Lysinhaltige Fermentationsbrühen mit einem pH-Wert von 4,5 und einem Bisulfitgehalt beschrieben werden.

In der EP 0 615 693 wird ein Verfahren zur Herstellung eines Tierfuttermittel-Additives auf FermentationsbrüheBasis offenbart, bei dem man die Fermentationsbrühe, ggf. nach Entfernung eines Teils der Inhaltsstoffe, zu einem Feinkorn, das zu mind. 70 Gew.-% eine maximale Partikelgröße von 100 $\mu$m hat, sprühtrocknet, und daß man dieses Feinkorn in einer zweiten Stufe zu einem Granulat aufbaut, das zu mind. 30 Gew.-% das Feinkorn enthält.

Gemäß GB 1 439 728 wird ein L-Lysin enthaltendes Konzentrat aus einer Fermentationsbrühe hergestellt, die man vor der Aufkonzentration mit HCl auf einen pH von ca. 6,4 ansäuert und der man zur Stabilisierung Bisulfit zusetzt.

Nach der Eindampfung wird weiter auf einen pH-Wert von 4,0 angesäuert und das gewünschte Produkt durch Sprühtrocknung erhalten.

Die EP-A 1 331 220 ($\cong$ US 2003/152633) betrifft granulierte Futtermitteladditive, die L-Lysin als Hauptkomponente enthalten.

Dort wurde gefunden, dass die Menge der Gegenionen für das Lysin wie z. B. die der Sulfationen verringert werden kann, indem man während der Fermentation entstehendes Kohlendioxid als Gegenion benutzt. Insgesamt wird ein Anionen/Lysin-Verhältnis von 0,68 bis 0,95 beansprucht.

Die Verringerung der Gegenionen wie z. B. Sulfat im L-Lysin enthaltenden Produkt soll zu einer Verbesserung der hygroskopischen Eigenschaften und der Verbackungsneigung führen.

Aufgabe der Erfindung

Aufgabe der Erfindung ist die Bereitstellung eines L-Lysin enthaltenden Futtermitteladditivs mit verbesserten Eigenschaften, hergestellt unter Verwendung der bei der Fermentation unter Verwendung von Ammoniumsulfat anfallenden Brühen, und eines Verfahrens mit geringerem Lysinverlust bei der Aufarbeitung der Brühen als nach dem Stand der Technik.

Beschreibung der Erfindung

Die Erfindung betrifft ein grenullertes Futtermitteladditiv auf Fermentationsbrühebasis mit

a) einem L-Lysingehalt von 10 bis 70 Gew.-% (ger. als L-Lysinbase), insbesondere 30 bis 60 Gew.-%,

b) einem Wassergehalt von 0,1 bis 5 Gew.-% Wasser, und

c) einem Sulfat/L-Lysinverhältnis von 0,85 bis 1,2 , bevorzugt 0,9 bis 1,0 , insbesondere >0,9 bis <0,95, wobei dieses Verhältnis errechnet wird nach der Formel

$$V = 2 \times [SO_4^{2-}] / [L\text{-Lysin}].$$

Die Differenz zu 100 Gew.-% wird durch weitere Bestandteile der Fermentationsbrühe und gegebenenfalls die Biomasse aufgefüllt.

Das Granulat weist einen pH-Wert von 3,5 bis 5,1 insbesondere 4,0 bis 5,0 bevorzugt 4,2 bis 4,8, gemessen in einer 10 Gew.-%igen Suspension in entionisiertem Wasser bei 25 °C mit einer pH-Elektrode, auf. Der Messwert stallt sich nach ca. 1 min konstant ein.

Das Molverhältnis von Sulfat zu L-Lysin wird im Anschluss an die Fermentation eingestellt, wobei eine $SO_4^{2-}$entheltende Verbindungen, insbesondere Ammoniumsulfat und Schwefelsäure in einer geeigneten Verdünnung zugesetzt wird.

Auf "Fermentationsbrühebasis" bedeutet, dass eine L-Lysin enthaltende Fermentationsbrühe ausgearbeitet wird, die die während der Fermentation entstandene Biomasse zu 0 bis 100 % enthält.

Gegenstand der Erfindung ist ebenfalls ein Verfahren zur Herstellung eines granulierten, L-Lysin enthaltenden Futtermittetadditivs durch Fermentation eines L-Lysin produzierenden Mikroorganismus in einem wäßrigen Medium unter Verwendung von Ammoniumsulfat und aeroben Bedingungen und Herstellen eines granulierten Produkts mit Hilfe von eigentlich bekannten Verfahren, wobei man nach Abschluss der Fermentation

a) gegebenenfalls das Verhältnis Sulfat/L-Lysin in der Fermentationsbrühe misst.

Die Sulfatkonzentration in der Fermentationsbrühe ist bekannt Das gilt im allgemeinen auch für die unter eingespielten Bedingungen erzeugten Mengen an L-Lysin, so dass die Messung in der Regel nur bei Schwankungen in der Produktion notwendig wird.

Das weitere Verfahren umfasst folgende Schritte:

a) Man setzt gegebenenfalls Ammoniumsulfat zu

b) Man senkt den pH-Wert durch Zugabe von Schwefelsäure auf 4,0 bis 5,2, insbesondere 4,9 bis 5,1 ab, wobei man durch die Zugabe der Sulfat-haltigen Verbindung (en) ein Sulfat/L-Lysin-Verhältnis von 0,85 bis 1,2 , bevorzugt 0,9 bis 1,0,insbesondere > 0,9 bis < 0,95 in der Brühe einstellt, und

c) man entzieht bevorzugt dem so erhaltenem Gemisch gegebenenfalls Wasser, granuliert und erhält ein Produkt mit einem L-Lysin-Gehalt von 10 bis 70 Gew.-% (ber. als Lysinbase, bezogen auf die Gesamtmenge) und einem Sulfat/L-Lysin-Verhältnis von 0,85 bis 1,2 , bevorzugt 0,9 bis 1,0 , insbesondere >0,9 bis <0,95, wobei das Verhältnis nach der Formel $2 \times [SO_4^{2-}] / [L\text{-Lysin}] = V$ berechnet wird.

Ein Verhältnis V von 1 bedeutet z. B., dass ein stöchiometrisch zusammengesetztes $(SO_4)$ $Lys_2$ vorliegt, während bei einem Verhältnis von 0,9 ein 10%iger Sulfatunterschuss gefunden wird.
Schritt b) kann auch vor Schritt a) durchgeführt werden.

In einer Variante beläuft sich der L-Lysingehalt auf 20 bis 65, insbesondere 30 bis 65 Gew.-%.

Es ist möglich, die Fermentation in Gegenwart einer solchen Menge von Ammoniumsulfat durchzuführen, dass nach Beendigung der Fermentation bereits ein Sulfat/Lysin-Verhältnis vorliegt, das im erfindungsgemäß beanspruchten Bereich liegt.

Die weitere Zugabe von Ammoniumsulfat ist dann gegebenenfalls nicht mehr notwendig.

Wird über die erfindungsgemäße pH-Wertabsenkung hinaus Säure zugesetzt, sind wegen der Pufferwirkung der in der Brühe enthaltenen Verbindungen erhöhte Mengen an Säure erforderlich, die dann zu einer unerwünschten Denaturierung und Auflösung der Zellen führen können.

Auch ohne Zusatz einer Säure sinkt der pH-Wert der Brühe bei der Aufkonzentration durch Eindampfen auf einen pH-Wert von ca. 5,4 ab.

Die erfindungsgemäß hergestellten Granulate haben einen pH-Wert von 3,5 bis 5,1 (gemessen in der Suspension, s. o.). Der Zusatz von Salzsäure wird bevorzugt ausgeschlossen. Ihr Anteil beläuft sich im allgemeinen auf max. 1%, insbesondere 0,01 bis 0,1 Gew.-%, bezogen auf die eingesetzte Schwefelsäure.

Sie weisen trotz des erhöhten Sulfatgehalts einen deutlich höheren Weissgrad, eine geringere Hygroskopizität und eine bessere Stabilität bei thermischer Belastung als Granulate mit demselben L-Lysingehalt auf, die suspendiert einen pH-Wert von 5,3 bis 5,7 und ein Sulfat/L-Lysin-Verhältnis im Bereich von z. B. 0,75 bis 0,87 zeigen, wie sie aus dem Stand der Technik bekannt sind (s. Beispiele).

Die genannten Eigenschaften sind durch den Zusatz von Ammonium-, Alkali-, oder Erdalkalisalzen der schwefeligen Säure oder deren Gemische, insbesondere Alkalihydrogensulfit bevorzugt Natriumhydrogensulfit in einer Menge von 0,01 bis 0,5, bevorzugt 0,1 bis 0,3, insbesondere 0,1 bis 0,2 Gew.-%, bezogen auf die Fermentationsbrühe weiter zu verbessern.

Die Sulfite, insbesondere Natriumhydrogensulfit werden bevorzugt als Lösung vor der Aufkonzentration der Fermentationsbrühe zugesetzt. Die eingesetzte Menge wird bevorzugt bei der Einstellung des Sulfat-/L-Lysinverhältnisses berücksichtigt.

Die Granulate sind z. B. herstellbar nach den Verfahren gemäß EP-B 0 615 693 oder EP-B 0 809 940, US 5 840 358 oder WO 2005/006875 oder WO 2004/054381. Sie besitzen im allgemeinen zu > 97 % eine mittlere Teilchengröße von $\geq$ 0,1 bis 1,8 mm und ein Schüttgewicht von 600 bis 950 kg/m$^3$ insbesondere 650 bis 900 kg/ m$^3$.

Die Farbwerte der Granulate liegen bevorzugt in den Bereichen:

ohne Hydrogensulfitzusatz: L* 65-70, a* 6-8, b* 20-25
mit Hydrogensulfitzusatz: L* >70-80, a* 4-<6, b* >25-30.

Das erfindungsgemäße Verfahren führt aber nicht nur zu Produkten mit vorteilhaften Eigenschaften.

Es zeigt sich gleichzeitig, dass der bei der Aufarbeitung von Fermentationsbrühe im allgemeinen auftretende Lysinverlust durch das Ansäuern im erfindungsgemäßen Umfang und Einstellung des Sulfat/Lysin-Verhältnisses vor der Aufkonzentration um ca. 50 % verringert werden kann.

Die Fermentation der erfindungsgemäß bevorzugt verwendeten coryneformen Bakterien, insbesondere der Gattung Corynebacterium glutamicum kann kontinuierlich - wie beispielsweise in der PCT/EP 2004/008882 beschrieben - oder diskontinuierlich im batch-Verfahren (Satzkultivierung) oder im fed batch (Zulaufverfahren) oder repeated fed batch Verfahren (repetitives Zulaufverfahren) zum Zwecke der Produktion von L-Lysin durchgeführt werden. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium beziehungsweise Fermentationsmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium und Fermentationsmedium beziehungsweise Medium sind gegenseitig austauschbar.

Als Kohlenstoffquelle können Zucker und Kohlehydrate wie z.B. Glucose, Saccharose, Laktose, Fructose, Maltose, Melasse, Saccharose-haltige Lösungen aus der Zuckerrüben- oder Zuckerrohrherstellung, Stärke, Stärkehydrolysat und Cellulose, Öle und Fette, wie zum Beispiel Sojaöl, Sonnenblumenöl, Erdnußöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumphosphat, Ammoniumcarbonat und Ammoniumnitrat, bevorzugt Ammoniumsulfat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium haltigen Salze verwendet werden.

Das Kulturmedium muß weiterhin Salze beispielsweise in Form von Sulfaten von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren beispielsweise Homoserin und Vitamine beispielsweise Thiamin, Biotin oder Pantothensäure zusätzlich zu den oben genannten Stoffen eingesetzt werden. Dem Kulturmedium können überdies geeignete Vorstufen der jeweiligen Aminosäure zugesetzt werden.

Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak beziehungsweise Ammoniakwasser oder saure Verbindungen wie Phosphorsäure oder Schwefelsäure in geeigneter Weise eingesetzt. Der pH wird im Allgemeinen auf einen Wert von 6,0 bis 9,0 vorzugsweise 6,5 bis 8 eingestellt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete selektiv wirkende Stoffe, wie zum Beispiel Antibiotika hinzugefügt werden. Um aerobe Bedingungen aufrechtzuerhalten, werden Sauerstoff oder Sauerstoff-haltige Gasmischungen, wie zum Beispiel Luft in die Kultur eingetragen. Die Verwendung von Flüssigkeiten, die mit Wasserstoffperoxid angereichert sind, ist ebenfalls möglich. Gegebenenfalls wird die Fermentation bei Überdruck, beispielsweise bei einem Druck von 0,03 bis 0,2 MPa, gefahren. Die Temperatur der Kultur liegt normalerweise bei 20 °C bis 45 °C und vorzugsweise bei 25 °C bis 40 °C. Bei batch-Verfahren wird die Kultivierung solange fortgesetzt, bis sich ein Maximum der gewünschten Aminosäure gebildet hat. Dieses Ziel wird normalerweise innerhalb von 10 Stunden bis 160 Stunden erreicht. Bei kontinuierlichen Verfahren sind längere Kultivierungszeiten möglich.

Beispiele für geeignete Fermentationsmedien finden sich unter anderem in den Patentschriften US 5, 770, 409, US 5,840,551 und US 5,990,350 oder US 5,275,940.

Methoden zur Bestimmung von L-Aminosäuren sind aus dem Stand der Technik bekannt. Die Analyse kann zum Beispiel so wie bei Spackman et al. (Analytical Chemistry, 30, (1958), 1190) beschrieben durch Anionenaustausch-Chromatographie mit anschließender Ninhydrin-Derivatisierung erfolgen, oder sie kann durch reversed phase HPLC erfolgen, so wie bei Lindroth et al. (Analytical Chemistry (1979) 51: 1167-1174) beschrieben.

Gegenstand der Erfindung ist dementsprechend ein Verfahren zur Herstellung einer L-Aminosäure bei dem man

a) ein coryneformes Bakterium in einem geeignetem Medium fermentiert und

b) die L-Aminosäure in der Fermentationsbrühe oder in den Zellen des isolierten coryneformen Bakteriums anreichert.

Die auf diese Weise hergestellte Fermentationsbrühe wird anschließend zu einem festen oder flüssigen Produkt weiterverarbeitet.

Unter einer Fermentationsbrühe versteht man ein Fermentationsmedium, in dem ein Mikroorganismus für eine gewisse Zeit und bei einer gewissen Temperatur kultiviert wurde. Das Fermentationsmedium beziehungsweise die während der Fermentation eingesetzten Medium enthält/enthalten sämtliche Substanzen beziehungsweise Komponenten, die eine Vermehrung des Mikroorganismus und eine Bildung der gewünschten Aminosäure sicherstellt.

Bei Abschluss der Fermentation enthält die entstandene Fermentationsbrühe dementsprechend a) die infolge der Vermehrung der Zellen des Mikroorganismus entstandene Biomasse des Mikroorganismus, b) das im Laufe der Fermentation gebildete L-Lysin, c) die im Laufe der Fermentation gebildeten organischen Nebenprodukte und d) die durch die Fermentation nicht verbrauchten Bestandteile des/der eingesetzten Fermentationsmediums/Fermentationsmedien beziehungsweise der Einsatzstoffe wie beispielsweise Vitamine wie Biotin, Aminosäuren wie Homoserin oder Salze wie Magnesiumsulfat.

Zu den organischen Nebenprodukte gehören Stoffe, die von den bei der Fermentation eingesetzten Mikroorganismen gegebenenfalls neben L-Lysin erzeugt und gegebenenfalls ausgeschieden werden. Hierzu zählen L-Aminosäuren, die im Vergleich zur erwünschten Aminosäure weniger als 30 %, 20 % oder 10 % ausmachen. Hierzu gehören weiterhin organische Säuren, die ein bis drei Carboxyl-Gruppen tragen wie zum Beispiel Essigsäure, Milchsäure, Zitronensäure, Apfelsäure oder Fumarsäure. Schließlich gehören dazu auch Zucker wie zum Beispiel Trehalose.

Typische für industrielle Zwecke geeignete Fermentationsbrühen haben einen L-Lysingehalt von 40 g/kg bis 180 g/kg oder 50 g/kg bis 150 g/kg. Der Gehalt an Biomasse (als getrocknete Biomasse) beträgt im Allgemeinen 20 bis 50 g/kg.

Bei Verfahren zur Herstellung von Lysin werden solche Verfahren bevorzugt, bei denen Produkte erhalten werden, die Bestandteile der Fermentationsbrühe enthalten. Diese werden insbesondere als Tierfuttermitteladditive verwendet.

Je nach Anforderung kann die Biomasse ganz oder teilweise durch Separationsmethoden wie z.B. der Zentrifugation, der Filtration, dem Dekantieren oder einer Kombination hieraus aus der Fermentationsbrühe entfernt oder vollständig in ihr belassen werden. Gegebenenfalls wird die Biomasse beziehungsweise die Biomasse enthaltene Fermentationsbrühe während eines geeigneten Verfahrensschrittes inaktiviert.

In einer Verfahrensweise wird die Biomasse vollständig oder nahezu vollständig entfernt, so dass keine (0 %) oder höchstens 30 %, höchstens 20 %, höchstens 10%, höchstens 5%, höchstens 1 % oder höchstens 0,1 % Biomasse im hergestellten Produkt verbleibt. In einer bevorzugten Verfahrensweise wird die Biomasse nicht oder nur in geringfügigen Anteilen entfernt, sodass sämtliche (100 %) oder mehr als 70 %, 80 %, 90 %, 95 %, 99 % oder 99,9 % Biomasse im hergestellten Produkt verbleibt.

Fermentationsbrühen, aus denen die Biomasse teilweise oder insgesamt entfernt wurde, können zur Standardisierung des Produkts eingesetzt werden. Dies gilt natürlich auch für die reinen Verbindungen L-Lysinbase und Lysinsulfat.

Erfindungsgemäß wird die nach der Fermentation erhaltene Fermentationsbrühe vor der Aufkonzentration mit Schwefelsäure angesäuert und gegebenenfalls mit Ammoniumsulfat versetzt. Schließlich kann die Brühe auch durch Zusatz von bevorzugt Natriumhydrogensulfit oder einem anderen Salz beispielsweise Ammonium-, Alkali- oder Erdalkalisalz der schwefligen Säure stabilisiert und aufgehellt werden.

Wird Biomasse abgetrennt, erfolgt dies bevorzugt vor der erfindungsgemäßen Absenkung des pH-Werts und der Zugabe von Ammoniumsulfat und Sulfitsalz.

Bei der gegebenenfalls vorgenommenen Abtrennung der Biomasse werden gegebenenfalls in der Fermentationsbrühe enthaltene organische oder anorganische Feststoffe teilweise oder ganz entfernt. Die in der Fermentationsbrühe gelösten organischen Nebenprodukte und die gelösten nicht verbrauchten Bestandteile des Fermentationsmediums (Einsatzstoffe) bleiben mindestens teilweise (> 0 %), bevorzugt zu mindestens 25 %, besonders bevorzugt zu mindestens 50 % und ganz besonders bevorzugt zu mindestens 75 % im Produkt. Gegebenenfalls bleiben diese auch vollständig (100 %) oder nahezu vollständig das heißt > 95 % oder > 98 % im Produkt. In diesem Sinne bedeutet der Begriff "Fermentationsbrühebasis", dass ein Produkt mindestens einen Teil der Bestandteile der Fermentationsbrühe enthält.

Anschließend wird der Brühe mit bekannten Methoden wie z.B. mit Hilfe eines Rotationsverdampfers, Dünnschichtverdampfers, Fallfilmverdampfers, durch Umkehrosmose oder durch Nanofiltration Wasser entzogen beziehungsweise eingedickt oder konzentriert. Diese aufkonzentrierte Fermentationsbrühe kann anschließend durch Methoden der Gefriertrocknung, der Sprühtrocknung, der Sprühgranulation oder durch anderweitige Verfahren wie zum Beispiel in der zirkulierenden Wirbelschicht gemäß PCT/EP 2004/006655 beschrieben, zu rieselfähigen Produkten insbesondere zu Granulat aufgearbeitet werden. Gegebenenfalls wird aus dem erhaltenen Granulat durch Sieben oder Staubabtrennung ein Produkt mit der gewünschten Korngröße isoliert.

Es ist ebenfalls möglich, ein feinteiliges Pulver direkt, d. h. ohne vorherige Aufkonzentrierung durch Sprühtrocknung oder Sprühgranulation der Fermentationsbrühe zu gewinnen.

Die Teilchengrößenbestimmung kann mit Methoden der Laserbeugungsspektrometrie durchgeführt werden. Die entsprechenden Methoden sind im Lehrbuch zur "Teilchengrößenmessung in der Laborpraxis" von R. H. Müller und R. Schuhmann, Wissenschaftliche Verlagsgesellschaft Stuttgart (1996) oder im Lehrbuch "Introduction to Particle Technology" von M. Rhodes, Verlag Wiley & Sons (1998) beschrieben.

Das rieselfähige, feinteilige Pulver kann wiederum durch geeignete Kompaktier- oder Granulier-Verfahren in ein grobkörniges, gut rieselfähiges, lagerbares und weitgehend staubfreies Produkt überführt werden.

Unter "rieselfähig" versteht man Pulver, die aus einer Serie von Glasauslaufgefäßen mit verschieden großen Auslauföffnungen mindestens aus dem Gefäß mit der Öffnung 5 mm (Millimeter) ungehindert auslaufen (Klein: Seifen, Öle, Fette, Wachse 94, 12 (1968)).

Mit "feinteilig" ist ein Pulver mit überwiegendem Anteil (> 50 %) einer Teilchengröße von 20 bis 200 $\mu$m Durchmesser gemeint.

Mit "grobkörnig" ist ein Produkt mit einem überwiegendem Anteil (> 50 %) einer Teilchengröße von 200 bis 2000 $\mu$m Durchmesser gemeint.

Der Begriff "staubfrei" bedeutet, daß das Produkt lediglich geringe Anteile (< 5 %) an Teilchengrößen unter 100 $\mu$m Durchmesser enthält.

Vorteilhaft bei der Granulation oder Kompaktierung ist der Einsatz von üblichen organischen oder anorganischen Hilfsstoffen, beziehungsweise Trägern wie Stärke, Gelatine, Cellulosederivaten oder ähnlichen Stoffen, wie sie üblicherweise in der Lebensmittel- oder Futterverarbeitung als Binde-, Gelier-, oder Verdickungsmittel Verwendung finden, oder von weiteren Stoffen wie zum Beispiel Kieselsäuren, Silikaten (EP-A 0 743 016) Stearaten.

Weiterhin ist es vorteilhaft die Oberfläche der erhaltenen Granulate mit Ölen zu versehen so wie es in der WO 04/054381 beschrieben ist. Als Öle können Mineralöle, pflanzliche Öle oder Mischungen pflanzlicher Öle verwendet werden. Beispiele für derartige Öle sind Sojaöl, Olivenöl, Sojaöl/Lecithingemische. In gleicherweise sind auch Silikonöle, Polyethylenglykole oder Hydroxyethycellulose geeignet. Durch die Behandlung der Oberflächen mit den genannten Ölen erzielt man eine erhöhte Abriebfestigkeit des Produktes und einen Verringerung des Staubanteils. Der Gehalt an Öl im Produkt beträgt 0,02 bis 2,0 Gew.-%, bevorzugt 0,02 bis 1,0 Gew.-%, und ganz besonders bevorzugt 0,2 bis 1,0 Gew.-% bezogen auf die Gesamtmenge des Futtermitteladditivs.

Bevorzugt sind Produkte mit einem Anteil von ≥ 97 Gew.-% einer Teilchengröße von ≥100 bis 1800 $\mu$m oder einem Anteil von ≥ 95 Gew.-% einer Teilchengröße von ≥ 300 bis 1800 $\mu$m Durchmesser. Der Anteil an Staub, d. h. Teilchen mit einer Korngrösse < 100 $\mu$m liegt bevorzugt bei > 0 bis 1 Gew.-% besonders bevorzugt bei maximal 0,5 Gew.-%.

Alternativ kann das Produkt aber auch auf einen in der Futtermittelverarbeitung bekannten und üblichen organischen oder anorganischen Trägerstoff wie zum Beispiel Kieselsäuren, Silikate, Schrote, Kleien, Mehle, Stärken Zucker oder andere aufgezogen und/oder mit üblichen Verdickungs- oder Bindemitteln vermischt und stabilisiert werden. Anwendungsbeispiele und Verfahren hierzu sind in der Literatur (Die Mühle + Mischfuttertechnik 132 (1995) 49, Seite 817) beschrieben.

Schließlich kann das Produkt auch durch Beschichtungsverfahren ("Coating") mit Filmbildnern wie beispielsweise Metallcarbonate, Kieselsäuren, Silikate, Alginate, Stearate, Stärken, Gummis und Celluloseether, wie in der DE-C 41 00 920 beschrieben, in einen Zustand gebracht werden, in dem es stabil gegenüber der Verdauung durch Tiermägen insbesondere dem Magen von Wiederkäuern ist.

Zur Einstellung einer gewünschten Aminosäurekonzentration im Produkt kann je nach Anforderung die entsprechende Aminosäure während des Verfahrens in Form eines Konzentrates oder gebenenfalls einer weitgehend reinen Substanz beziehungsweise dessen Salz in flüssiger oder fester Form hinzugefügt werden. Diese können einzeln oder als Mischungen zur erhaltenen oder aufkonzentrierten Fermentationsbrühe, oder auch während des Trocknungs- oder Granulationsprozesses hinzugefügt werden.

Im Falle des Lysin hat das auf diese Weise hergestellte feste Produkt auf Fermentationsbrühebasis einen Lysingehalt (gerechnet als Lysinbase) von 10 Gew.-% bis 70 Gew.-%, bevorzugt 30 Gew.-% bis 60 Ges.-% und ganz besonders bevorzugt von 40 Gew.-% bis 60 Gew.-% bezogen auf die Gesamtmenge des Produkts.

In den Untersuchungen konnte gezeigt werden, dass die Einstellung des pH-Wertes in der Fermentationsbrühe auf Werte $\geq$ pH 5,2, die Erhöhung des Sulfat/Lysinverhältnisses und bevorzugt eine Sulfitzugabe im Bereich von 0,01 bis 0,5 Gew.-% in der Fermentationsbrühe nach der Fermentation den Lysinverlust während der Aufarbeitung der Fermentationsbrühe deutlich reduziert.

Dabei führt die Kombination der verschiedenen Maßnahmen vor der Aufarbeitung zu einem synergistischen Effekt im Vergleich zur Summe der Einzeleffekte.

In nicht behandelten Fermentationsbrühen (keine Zugabe eines der Additive) resultiert bei der Einengung zum Konzentrat ein mittlerer Lysinverlust von ca. 3,5 Gew.-% (ohne Granulationsschritt). Die Erhöhung des Sulfatverhältnisses durch Zugabe von Ammoniumsulfat führt am Ende zu einem mittleren Lysinverlust von ca. 3,2 Gew.-%, die alleinige pH-Werteinstellung reduziert den mittleren Lysinverlust auf ca. 1,4 Gew.-%.

Die Kombination von pH-Werteinstellung und Erhöhung des Sulfatverhältnisses zeigt eine höhere Schutzwirkung für das Lysin und resultiert in einem mittleren Lysinverlust von ca. 0,9 Gew.-%. Die Kombination von pH-Werteinstellung und Natriumhydrogensulfitzugabe ergibt zusammen mit der Kombination aller drei Additive nur einen mittleren Lysinverlust von noch ca. 0,6 Gew.-% bzw. ca. 0,7 Gew.-%.

Er ist somit bei der Berechnung des Sulfat/L-Lysin-Verhältnisses im allgemeinen vernachlässigbar.

Somit konnte eindeutig gezeigt werden, dass die Vorbehandlung von Lysin-haltiger Fermentationsbrühe durch Absenkung des pH-Wertes, Erhöhung der Sulfatbilanz und Zugabe von Sulfit einen Schutzeffekt auf das vorhandene Lysin ausübt. Weiterhin wird die Hellfärbung des Produkts und die Stabilität bei thermischer Belastung verbessert.

Diese Vorteile bleiben bei der Weiterverarbeitung des Konzentrats zum Granulat weitgehend erhalten.

1. Versuchsdurchführung

1.1 Fermentation

Es wurden Fermentationen gemäß EP 0 533 039 durchgeführt. Die Granulate wurden daraus gemäß dem in der EP-B 0 809 940 beschriebenen Verfahren hergestellt. Die so hergestellten Granulate A bis D wurden mit den erfindungsgemäß hergestellten Granulaten E und F verglichen. Der L-Lysingehalt der Proben wurde standardisiert und auf ca. 51 bis 52 % eingestellt.

1.2 Farbmessung

Die L*a*b*-Farbmessung wurde wie folgt ausgeführt:

Prinzip

Das 3-Bereichs-Farbmessgerät zur Farb- und Remissionsmessung arbeitet nach dem in DIN 5033 beschriebenen Prinzip, wobei die diffuse Reflexion der Probe unter einem Winkel von 8° gemessen wird. Das reflektierte Licht wird dabei über einen Lichtleiter in das Gerät zur Aufspaltung auf die exakt definierten Normfarbfilter übertragen. Gemessen wird gegen einen Kalibrierstandard.

Ausrüstung:

Farbmessgerät Micro Color II LMC (Hersteller Fa. Dr. Lange)
Micro Color II Labor-Station LDC 20
Weißstandard LZM 076
Positionierkappe
Lichtschutzkappe 0 50 mm
Pulverküvette 0 34 mm

Durchführung

• Microcolor II kalibrieren (gemäß Bedienungsanleitung)

• Auswerteprogramm auswählen ($\rightarrow$ L*a*b*)

• nach Kalibrierung Positionierkappe aufsetzen

• Produkt zu zwei Drittel locker in die saubere Küvette einfüllen

• Produkt vorsichtig einrütteln, um eine gleichmäßige Befüllung zu erreichen

• Küvettenboden mit einem weichen Tuch reinigen

• die Küvette auf die Messöffnung setzen und mit der Lichtschutzkappe abdecken

• messen

Anmerkung

Bei der Messung pulverförmiger Substanzen ist auf eine gleichmäßige Komgröße (möglichst klein) zu achten. Bei grobkörnigen Substanzen wird eine Zweifachmessung durchgeführt.
Erläuterungen des L*a*b*-Systems:

L*: schwarz-weiß-Bereich
a*: rot-grün-Bereich
b*: gelb-blau-Bereich

1.2.1 Farbmessung an Produkten aus Vergleichsversuchen und gemäß Erfindung

Tabelle 1

|   | Lysin [%] | pH (10% in wasser) | [%] L* | [%] a* | [%] b* | Sulfat/Lysinverh. |
|---|-----------|--------------------|--------|--------|--------|-------------------|
| A | 51,4 | 5,6 | 60 | 9 | 16 | 0,83 |
| B | 52,3 | 5,7 | 62 | 8 | 17 | 0,82 |
| C | 51,3 | 5,6 | 60 | 8 | 19 | 0,84 |
| D | 52,5 | 5,3 | 61 | 9 | 21 | 0,83 |
| E | 51,6 | 4,6 | 67 | 8 | 25 | 0,9 |
| F | 52,5 | 4,5 | 76,0 | 5,0 | 26,0 | 0,95 |

In Tabelle 1 sind die Ergebnisse der Farbbestimmung für die Vergleichsversuche A bis D aufgeführt. Auch ohne Ansäuern der Fermentationsbrühe erhält man Produkte mit saurem pH-Wert, deren Farbwerte aber nicht die der erfindungsgemäßen Produkte erreichen.

Die Proben E und F entsprechen den erfindungsgemäßen Produkten, die nach der Ansäuerung der Brühe auf pH 5,1 erhalten wurden, wobei der zur Herstellung von Probe F eingesetzten Brühe zusätzlich 0,2 Gew.-% Natriumbisulfit zugesetzt wurden.

Es zeigt sich, dass die Proben E und F deutlich heller sind als die Produkte nach dem Stand der Technik. Das Sulfat/ Lysinverhältnis wird nach der Formel 2 x [$SO_4^{2-}$] / [L-Lysin]= Verhältnis bestimmt.

1.3 Produktstabilität nach thermischer Belastung

Tabelle 2 zeigt die Überlegenheit der erfindungsgemäßen Produkte E und F bei thermischer Belastung im Hinblick auf einen geringeren L-Lysinveriust.

1.4 Wasseraufnahme (Hygroskopizität)

1.4.1 Messung der Wasseraufnahme (Hygroskopitäts-Test)

Prinzip: Um die Wasseraufnahme der zu prüfenden Substanz zu bestimmen wird diese über einen definierten Zeitraum einem definierten Standardklima von 40°C und 75% rel. Luftfeuchte (entsprechend ICH) ausgesetzt. Die Wasseraufnahme wird gravimetrisch bestimmt.

Ausrüstung: Klimakammer, Standardklima 40°C/75% rel. Feuchte, flache Wägegegläschen mit Glasdeckel, (Durchmesser ca. 5 cm) Analysenwaage (Ablesbarkeit 0,0001 g).

Durchführung:

• Tara des Wägegläschen mit Deckel bestimmen

• 5 g der zu bestimmenden, homogenen Substanz genau einwiegen

• offenes Wägegläschen unter folgenden Bedingungen in der Klimakammer lagern:

| | |
|---|---|
| Temparatur | = 40°C |
| LF, rel. | = 75% |
| Zeit | = 1 h, 4 h |

• verschlossenes Wägegläschen nach 1 h und 4 h auswiegen und die Wasseraufnahme berechnen. Berechnung:

$$\text{Wasseraufnahme:} \quad \frac{((A-T)-E) \times 100}{E} \quad [\%]$$

A = Auswaage nach 1 h, 4h in g
T = Tara Wägegläschen mit Deckel in g
E = Einwaage der Probe in g

Anmerkung: Möchte man die Wasseraufnahme über einen längeren Zeitraum verfolgen, wird eine Wasseraufnahmekurve ermittelt. Dazu erfolgt die Auswaage der bewitterten Probe in den ersten 6 h stündlich und einmal nach 24h.

Graphische Darstellung:

| | |
|---|---|
| x-Achse: | Zeit in h |
| y-Achse: | Wasseraufnahme in % |

1.4.2 Wasseraufnahme von Produkten aus Vergleichsversuchen und gemäß Erfindung

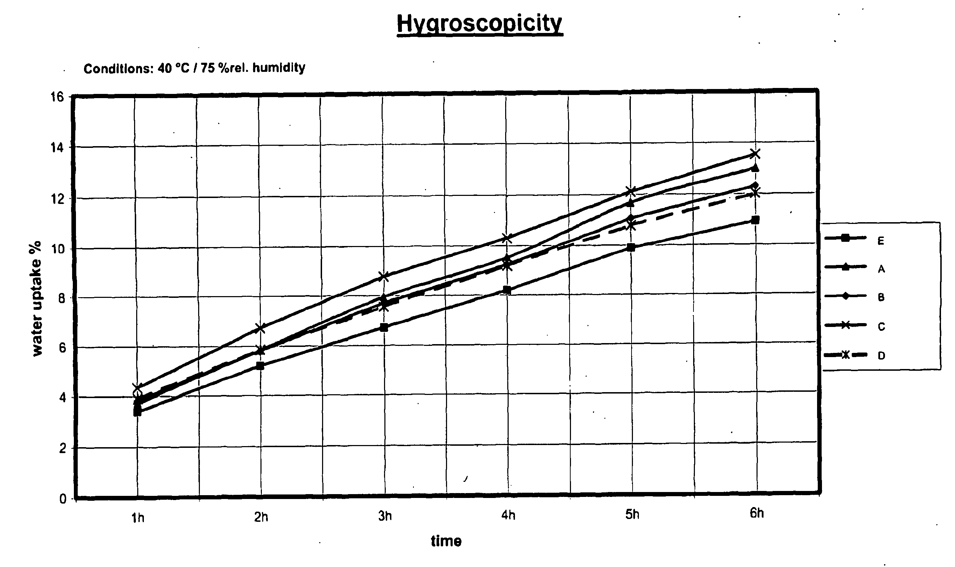

Abbildung 1 zeigt, dass das erfindungsgemäße Produkt E geringere Wassermengen/Zeit aufnimmt als die Produkte A bis D nach dem Stand der Technik. Eine geringere Hygroskopizität ist für die Verarbeitbarkeit von grosser Bedeutung.

Tabelle 2:

| | | A | B | D | E | F |
|---|---|---|---|---|---|---|
| | Stabilität bei thermischer Belastung (85°C). | | | | | |
| | | Lys (%) | Lys (%) | Lys (%) | Lys (%) | Lys (%) |
| 85°C | Initial | 51,4 | 52,3 | 52,5 | 51,6 | 52,2 |
| | 1 Woche | 48,4 | 49,4 | 49,1 | 50,8 | 52,0 |
| | 2 Wochen | 48,5 | 48,1 | 47,1 | 50,3 | 51,2 |
| | 3 Wochen | 48,2 | 46,8 | 47,3 | 49,8 | 51,1 |
| | diff.%abs | -3,2 | -5,5 | -5,2 | -1,8 | -1,4 |

1. Herstellversuche

Die Fermentionen wurden entsprechend der EP-B 0 533 039 durchgeführt.
Die Granulation erfolgte gemäß EP-B 0 809 940 (US 5,840,358).

2.1 Vergleichsversuch, Stand der Technik

Es wurde, wie in der EP 0 533 039 beschrieben, fermentiert und keine Biomasse abgetrennt. Es ergaben sich folgende Werte (L-Lysingehalt ist gerechnet als Gehalt an Lysin-Base in der Trockenmasse):

Tabelle 3

| L-Lysingehalt (Gew.-%) (ohne Wasser) | pH | Sulfat/Lysin-Verhältnis |
|---|---|---|
| 57,2 | 7,8 | 0,82 |

100 kg der Fermentationsbrühe wurden auf 65 °C erhitzt, in einen Verdampfer überführt und dort bei 82 °C und -0,5 bar Vakuum aufkonzentriert.
Die aufkonzentrierte Brühe wurde gemäß EP-B 0 809 940 granuliert.
Es ergab sich ein L-Lysinverlust von 5,1 Gew.-%.

Tabelle 4

| L-Lysingehalt(Gew.-%)(ohneWasser) | Wasser (%) |
|---|---|
| 52,1 | 2 |

2.2 Zusatz von Ammoniumsulfat und Schwefelsäure

Die Spezifikation der Fermentationsbrühe war:

Tabelle 5

| L-Lysingehalt (Gew.-%) (ohne Wasser) | pH | Sulfat/Lysin-Verhältnis |
|---|---|---|
| 57,7 | 7,5 | 0,9 |

1,35 kg Ammoniumsulfatlösung (37 % Festanteil) wurden zu 100 kg der Fermentationsbrühe hinzugefügt, so dass sich das Sulfat/Lysinverhältnis auf 0,93 erhöhte.
Der pH-Wert wurde durch Zugabe von 0,54 kg Schwefelsäure (ca. 93 %ig) auf pH 5,2 abgesenkt, so dass der anfängliche L-Lysingehalt von 57,7 % auf 55,7 Gew.-% absinkt.

Tabelle 6

| L-Lysingehalt (Gew.-%) (ohne Wasser) | pH | Sulfat/Lysin-Verhältnis |
|---|---|---|
| 55,7 | 5,2 | 0,93 |

Die erhaltene Fermentationsbrühe wurde auf 55 °C erhitzt und dann wie in Beispiel 2.1 aufkonzentriert und granuliert.

Es ergab sich ein Verlust von 3,3 Gew.-% und damit eine Verbesserung von ca. 35 % gegenüber dem Vergleichsversuch.

Tabelle 7

| L-Lysingehalt (Ges.-%) (ohne Wasser) | Wasser (%) |
|---|---|
| 52,4 | 2,55 |

2.3 Zusatz von Ammoniumsulfat, Schwefelsäure und Natriumhydrogensulfit

Die Spezifikation der eingesetzten Fermentationsbrühe war:

Tabelle 8

| L-Lysingehalt (Gew.-%) (ohne Wasser) | pH | Sulfat/Lysin-Verhältnis |
|---|---|---|
| 57,3 | 7,8 | 0,95 |

DasSulfat/L-Lysin-Verhältnis belief sich nach der Fermentation auf 0,95, so dass kein weiteres Ammoniumsulfat mehr zudosiert wurde.

Es wurden 0,105 kg Natriumhydrogensulfit zu 100 kg der Fermentationsbrühe zugesetzt, 30 min gerührt und anschließend 0,61 kg Schwefelsäure zugemischt, wobei sich ein pH-Wert von 5,2 einstellte.

Durch die Zugabe des Hydrogensulfits sinkt der L-Lysingehalt auf 57,0 Gew.-% und durch den Säurezusatz aufgrund des Verdünnungseffekts weiter auf 55,1 Gew.-%, die Menge der Trockenmasse nimmt zu.

Die Spezifikation der Fermentationsbrühe war:

Tabelle 9

| L-Lysingehalt (Gew.-%) (ohne Wasser) | pH | Sulfat/Lysin-Verhältnis |
|---|---|---|
| 55,1 | 5,2 | 0,96 |

Die erhaltene Fermentationsbrühe wurde auf 55 °C erhitzt und dann wie in Beispiel 2.1 aufkonzentriert und granuliert.

Tabelle 10

| L-Lysingehalt (Gew.-%) (ohne Wasser) | Wasser (%) |
|---|---|
| 53,0 | 1,9 |

Es ergab sich ein L-Lysinverlust von 2,1 Gew.-% und damit eine Verbesserung um fast 60 % gegenüber dem Vergleichsversuch.

**Patentansprüche**

**1.** Granuliertes Futtermitteladditiv mit verbessertem Farbwert auf Fermentationsbrühebasis mit

a) einem L-Lysingehalt von 10 bis 70 Gew.-%, insbesondere 30 bis 60 Gew.-% (ger. als Base, bezogen auf das Gesamtgewicht)

b) einem Wassergehalt von 0,1 bis 5 Gew.-% bezogen auf das Gesamtgewicht,

c) einem Sulfat/L-Lysinverhältnis von 0,85 bis 1,2, gerechnet nach der Formel 2 x $[SO_4^{2-}]$ / [L-Lysin] = Verhältnis, und

d) einem pH-Wert von 3,5 bis 5,1 gemessen in einer 10 Gew.-%igen Suspension in entionisiertem Wasser bei 25 °C mit einer pH-Elektrode.

2. Futtermitteladditiv gemäß Anspruch 1, das zu > 97 % eine mittlere Teilchengröße von ≥ 0,1 bis 1,8 mm besitzt.

3. Futtermitteladditiv gemäß Anspruch 1 oder 2, dessen Oberfläche mit einem Öl in einer Menge von 0,02 bis 2 Gew.-%, bezogen auf die Gesamtmenge des Futtermitteladditivs, belegt ist.

4. Futtermitteladditiv gemäß den Ansprüchen 1 bis 3, dessen Farbwerte in folgenden Bereichen liegen (Messung der diffusen Reflexion der Probe unter einem Winkel von 8°):

L* 65-80 (Schwarz-weiß-Bereich)
a* 4-8 (Rot-grün-Bereich)
b* 20-30 (Gelb-blau-Bereich).

5. Verfahren zur Herstellung eines granulierten, L-Lysin enthaltenden Futtermitteladditivs durch Fermentation eines L-Lysin produzierenden Mikroorganismus in einem wäßrigen Medium unter aeroben Bedingungen, wobei man nach Abschluss der Fermentation

a) gegebenenfalls das Sulfat/L-Lysin-Verhältnis in der Fermentationsbrühe bestimmt,

b) anschließend gegebenenfalls Ammoniumsulfat zusetzt und

c) den pH-Wert durch Zugabe von Schwefelsäure auf 4,9 bis 5,2 absenkt, wobei man durch die Zugabe der Sulfat-haltigen Verbindung(en) ein Sulfat/L-Lysin-Verhältnis von 0,85 bis 1,2 in der Brühe einstellt,

d) das so erhaltene Gemisch granuliert und ein Produkt mit einem L-Lysin-Gehalt von 10 bis 70 Gew.-% (bestimmt als Lysinbase, bezogen auf die Gesamtmenge) erhält.

6. Verfahren gemäß Anspruch 5, bei dem man die Brühe zwischen den Schritten c) und d) durch Wasserentzug aufkonzentriert

7. Verfahren gemäß Anspruch 5, bei dem man die Reihenfolge der Zugaben von Ammoniumsulfat und Schwefelsäure umkehrt.

8. Verfahren gemäß den Ansprüchen 5, 6 oder 7, bei dem man vor der Aufkonzentration ein Ammonium-, Erdalkali- oder Alkalihydrogensulfit in einer Menge von 0,01 bis 0,5 Gew.-%, bezogen auf Fermentationsbrühe, zusetzt.

9. Verfahren gemäß den Ansprüchen 5 bis 8, bei dem man nach Abschluss der Fermentation 0 bis 100 % der während der Fermentation gebildeten Biomasse entfernt und die Schritte a) bis d) anschließt.

10. Verfahren gemäß den Ansprüchen 5, 6 oder 7, bei dem man die Oberfläche des Granulats mit einem Öl in einer Menge von 0,02 bis 2 Gew.-%, bezogen auf die Gesamtmenge des Futtermitteladditivs, belegt.

11. Verfahren gemäß den Ansprüchen 5 bis 9, bei dem man coryneforme Mikroorganismen einsetzt.

12. Verfahren gemäß den Ansprüchen 5 bis 10, bei dem man Corynebakterien einsetzt.

**Claims**

1. Granulated fermentation-broth-based feed additive with an improved colour value having

a) an L-lysine content of 10 to 70% by weight (calculated as base, based on the total weight), in particular 30 to 60% by weight,

b) a water content of 0.1 to 5% by weight based on the total weight,

c) a sulphate/L-lysine ratio of 0.85 to 1.2, this ratio being calculated according to the formula 2 x $[SO_4^{2-}]$ / [L-lysine] = ratio, and
d) a pH of 3.5 to 5.1, measured in a 10% strength by weight suspension in deionized water at 25°C using a pH electrode.

**2.** Feed additive according to Claim 1, up to > 97% of which has a mean particle size of $\geq$ 0.1 to 1.8 mm.

**3.** Feed additive according to Claim 1 or 2, the surface of which is coated with an oil in an amount of 0.02 to 2% by weight, based on the total amount of the feed additive.

**4.** Feed additive according to Claims 1 to 3, the colour values of which are in the following ranges (measurement of the diffuse reflection of the sample at an angle of 8°):

L* 65-80 (black-white range)
a* 4-8 (red-green range)
b* 20-30 (yellow-blue range).

**5.** Method for producing a granulated L-lysine-containing feed additive by fermentation of an L-lysine-producing microorganism in an aqueous medium under aerobic conditions, wherein, after completion of the fermentation

a) the sulphate/L-lysine ratio in the fermentation broth is determined, if appropriate,
b) subsequently if appropriate ammonium sulphate is added and
c) the pH is lowered by adding sulphuric acid to 4.9 to 5.2, a sulphate/L-lysine ratio of 0.85 to 1.2 being set in the broth by the addition of the sulphate-containing compound(s), and
d) the resultant mixture is granulated and a product is obtained which has an L-lysine content of 10 to 70% by weight (determined as lysine base, based on the total amount).

**6.** Method according to Claim 5, in which the broth is concentrated between steps c) and d) by water removal.

**7.** Method according to Claim 5, in which the sequence of the additions of ammonium sulphate and sulphuric acid is reversed.

**8.** Method according to Claims 5, 6 or 7, in which, before the concentration, an ammonium, alkaline earth metal or alkali metal hydrogensulphite is added in an amount of 0.01 to 0.5% by weight, based on fermentation broth.

**9.** Method according to Claims 5 to 8, in which, after completion of the fermentation, 0 to 100% of the biomass formed during the fermentation is removed and steps a) to d) follow.

**10.** Method according to Claims 5, 6 or 7, in which the surface of the granules is coated with an oil in an amount of 0.02 to 2% by weight, based on the total amount of the feed additive.

**11.** Method according to Claims 5 to 9, in which coryneform microorganisms are used.

**12.** Method according to Claims 5 to 10, in which corynebacteria are used.

**Revendications**

**1.** Additif granulé pour aliment pour animaux, à valeur chromatique améliorée, à base de bouillon de fermentation, ayant

a) une teneur en L-lysine de 10 à 70 % en poids, en particulier de 30 à 60 % en poids (calculée en tant que base, par rapport au poids total)
b) une teneur en eau de 0,1 à 5 % en poids, par rapport au poids total,
c) un rapport sulfate/L-lysine de 0,85 à 1,2, calculé selon la formule 2 x $[SO_4^{2-}]$ / [L-lysine] = rapport, et
d) un pH de 3,5 à 5,1, mesuré à l'aide d'une électrode à pH dans une suspension à 10 % en poids dans de l'eau désionisée, à 25°C.

**2.** Additif pour aliment pour animaux selon la revendication 1, qui présente à raison de > 97 % une taille moyenne de particule de ≥ 0,1 à 1,8 mm.

3. Additif pour aliment pour animaux selon la revendication 1 ou 2, dont la surface est recouverte d'une huile en une quantité de 0,02 à 2 % en poids, par rapport à la quantité totale de l'additif pour aliment pour animaux.

4. Additif pour aliment pour animaux selon les revendications 1 à 3, dont les coordonnées chromatiques se situent dans les plages suivantes (mesure de la réflexion diffuse de l'échantillon sous un angle de 8°) :

   L* 65-80 (plage noir-blanc)
   a* 4-8 (plage rouge-vert)
   b* 20-30 (plage jaune-bleu).

5. Procédé pour la préparation d'un additif granulé pour aliment pour animaux, contenant de la L-lysine, par culture par fermentation d'un micro-organisme produisant de la L-lysine, dans un milieu aqueux, dans des conditions aérobies, dans lequel, une fois la fermentation terminée,

   a) éventuellement on détermine le rapport sulfate/L-lysine dans le bouillon de fermentation,
   b) ensuite éventuellement on ajoute du sulfate d'ammonium et
   c) on abaisse le pH à 4,9-5,2 par addition d'acide sulfurique
   en ajustant par l'addition du/des composé(s) contenant du sulfate un rapport sulfate/L-lysine de 0,85 à 1,2 dans le bouillon,
   d) le mélange ainsi obtenu est granulé et on obtient un produit ayant une teneur en L-lysine de 10 à 70 % en poids (déterminé en tant que base lysine base, par exemple à la quantité totale).

6. Procédé selon la revendication 5, dans lequel entre les étapes c) et d) on concentre le bouillon par déshydratation.

7. Procédé selon la revendication 5, dans lequel on inverse l'ordre des additions de sulfate d'ammonium et d'acide sulfurique.

8. Procédé selon la revendication 5, 6 ou 7, dans lequel, avant la concentration, on ajoute un hydrogénosulfite d'ammonium, de métal alcalin ou de métal alcalino-terreux, en une quantité de 0,01 à 0,5 % en poids, par rapport au bouillon de fermentation.

9. Procédé selon les revendications 5 à 8, dans lequel, une fois la fermentation terminée, on élimine 0 à 100 % de la biomasse formée pendant la fermentation et on effectue ensuite les étapes a) à d).

10. Procédé selon la revendication 5, 6 ou 7, dans lequel on recouvre la surface du produit granulé avec une huile en une quantité de 0,02 à 2 % en poids, par rapport à la quantité totale de l'additif pour aliment pour animaux.

11. Procédé selon les revendications 5 à 9, dans lequel on utilise des micro-organismes corynéformes.

12. Procédé selon les revendications 5 à 10, dans lequel on utilise des corynébactéries.

**Hygroscopicity**

Conditions: 40 °C / 75 % rel. humidity

E
A
B
C
D

water uptake %

16
14
12
10
8
6
4
2
0

1h  2h  3h  4h  5h  6h

time

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0533039 A
- GB 1439121 A
- EP 0615693 A
- GB 1439728 A
- EP 1331220 A
- US 2003152633 A
- EP 0615693 B
- EP 0809940 B
- US 5840358 A
- WO 2005006875 A
- WO 2004054381 A
- EP 2004008882 W
- US 5770409 A
- US 5840551 A
- US 5990350 A
- US 5275940 A
- EP 2004006655 W
- EP 0743016 A
- WO 04054381 A
- DE 4100920 C
- EP 0533039 B

**In der Beschreibung aufgeführte Nicht-Patentliteratur**

- **CHMIEL.** Bioprozesstechnik 1. Einführung in die Bioverfahrenstechnik. Gustav Fischer Verlag, 1991
- **STORHAS.** Bioreaktoren und periphere Einrichtungen. Vieweg Verlag, 1994
- Manual of Methods for General Bacteriology. American Society for Bacteriology, 1981
- **SPACKMAN et al.** *Analytical Chemistry,* 1958, vol. 30, 1190
- **LINDROTH et al.** *Analytical Chemistry,* 1979, vol. 51, 1167-1174
- **R. H. MÜLLER ; R. SCHUHMANN.** Teilchengrößenmessung in der Laborpraxis. Wissenschaftliche Verlagsgesellschaft, 1996
- **M. RHODES.** Introduction to Particle Technology. Verlag Wiley & Sons, 1998
- *Klein: Seifen, Öle, Fette, Wachse,* 1968, vol. 94, 12